# EUROPÄISCHE PATENTSCHRIFT

(11) **EP 4 200 608 B1**
(45) Veröffentlichungstag und Bekanntmachung des Hinweises auf die Patenterteilung: **10.09.2025**
(21) Anmeldenummer: 21791277.3
(22) Anmeldetag: 06.10.2021
(51) Int. Cl.: G01N 33/28, B01D 19/00

(54) **VORRICHTUNG UND VERFAHREN ZUM ENTGASEN EINER EINRICHTUNG UND ENTSPRECHENDES PRÜFSYSTEM ZUR GASANALYSE**
APPARATUS AND METHOD FOR DEGASSING A DEVICE, AND CORRESPONDING TEST SYSTEM FOR GAS ANALYSIS
APPAREIL ET PROCÉDÉ DE DÉGAZAGE D'UN DISPOSITIF, ET SYSTÈME DE TEST CORRESPONDANT POUR L'ANALYSE DES GAZ

(30) Priorität: 19.10.2020 AT 508952020
(43) Veröffentlichungstag der Anmeldung: 28.06.2023
(73) Patentinhaber: Omicron electronics GmbH, 6833 Klaus (AT)
(72) Erfinder: GISELBRECHT, Dietmar, 6900 Bregenz (AT); ANGLHUBER, Martin, 6800 Feldkirch (AT)
(74) Vertreter: Kraus & Lederer PartGmbB
(86) Internationale Anmeldenummer: PCT/EP2021/077570
(87) Internationale Veröffentlichungsnummer: WO 2022/084042

(56) Entgegenhaltungen:
- CN-U- 210 674 325
- DE-A1- 102017 126 136
- US-A- 4 407 665
- US-A- 4 456 172
- US-A- 4 763 514

## Beschreibung

### GEBIET DER ERFINDUNG

Die vorliegende Erfindung betrifft eine Vorrichtung und ein Verfahren zum Entgasen einer Einrichtung, wobei extrahierte Gase komprimiert werden, um beispielsweise gelöste Gase besser analysieren zu können.

### HINTERGRUND DER ERFINDUNG

Die DE 10 2017 126 136 A1 betrifft eine Vorrichtung zur Entgasung von Flüssigkeiten umfassend ein Entgasungssystem, wobei das Entgasungssystem eine erste Entgasungskammer, eine zweite Entgasungskammer, einen Flüssigkeitsvorrat, eine Pumpe sowie eine Zufuhrleitung aufweist, die den Flüssigkeitsvorrat mit der ersten Entgasungskammer verbindet, wobei die Pumpe saugseitig mit der ersten Entgasungskammer und druckseitig mit der zweiten Entgasungskammer verbunden ist, wobei das Entgasungssystem des Weiteren eine absperrbare Rückführungsleitung aufweist, die die beiden Entgasungskammern miteinander verbindet, und wobei die Vorrichtung einen Kontroller umfasst, der ausgebildet ist, das Entgasungssystem in einem ersten Betriebsmodus und in einem zweiten Betriebsmodus zu betreiben, wobei in dem ersten Betriebsmodus das Entgasungssystem derart geschaltet ist, dass die Pumpe Flüssigkeit aus der ersten Entgasungskammer abführt und einer Abnahmeeinheit für entgaste Flüssigkeit zuführt, und wobei das Entgasungssystem in dem zweiten Betriebsmodus derart geschaltet ist, dass Flüssigkeit durch die Rückführungsleitung aus der zweiten in die erste Entgasungskammer rückgeführt wird.

Beispielsweise zur Zustandsbestimmung und Fehlererkennung von Öl-Papier-isolierten Leistungstransformatoren ist die Analyse von in einem Isolieröl gelösten Gasen (Dissolved Gas Analysis (DGA)) ein wichtiges Vorgehen. Dabei müssen die zu analysierenden Gase vor der Analyse z.B. aus dem Isolieröl gelöst werden, was als Entgasung bekannt ist. Eine solche Entgasung kann durch unterschiedliche Verfahren erfolgen. Die höchste Extraktionsrate wird dabei mit einer vollständigen Entgasung mittels Vakuum erzielt.

Die Extraktion des Gases z.B. aus dem Isolieröl ist umso effektiver, je geringer der Druck in dem das Gas aufnehmenden Gefäß bzw. Volumen ist. Auch Entgasungspumpen, mit denen eine solche Extraktion des Gases vorgenommen wird, arbeiten umso effizienter je niedriger der Differenzdruck zwischen dem Druck eingangs der Pumpe und dem Druck ausgangs der Pumpe ist. Daher wird das Volumen auf der Ausgangsseite der Pumpe nach dem Stand der Technik so gewählt, dass der Druck in diesem Volumen bei der Entgasung oder Extraktion nicht zu sehr ansteigt.

Allerdings benötigen zahlreiche Analyseverfahren eine möglichst hohe Konzentration an Gas, um bestimmte Gaskomponenten oder bestimmte Gase, welche in dem extrahierten Gas enthalten sind, möglichst gut detektieren zu können.

### ZUSAMMENFASSUNG DER ERFINDUNG

Daher stellt sich die vorliegende Erfindung die Aufgabe, bei der Entgasung mit einem geringen Druck des extrahierten Gases zu arbeiten, während bei der Analyse des extrahierten Gases mit einem hohen Druck der extrahierten Gase gearbeitet wird. Dabei sollen die Mittel zur Lösung dieses vermeintlichen Widerspruchs möglichst preiswert sein und möglichst wenig Platz benötigen.

Erfindungsgemäß wird diese Aufgabe durch eine Vorrichtung zur Entgasung einer Einrichtung nach Anspruch 1, durch ein Prüfsystem nach Anspruch 10 und durch ein Verfahren zur Entgasung einer Einrichtung nach Anspruch 11 gelöst. Die abhängigen Ansprüche definieren bevorzugte und/oder vorteilhafte Ausführungsformen der vorliegenden Erfindung.

Im Rahmen der vorliegenden Erfindung wird eine Vorrichtung zur Entgasung einer Einrichtung (insbesondere eines Entgasungsgefäßes) bereitgestellt. Diese Vorrichtung umfasst eine Steuereinrichtung, eine Pumpe, ein erstes Volumen oder Gefäß, ein zweites Volumen oder Gefäß, ein erstes Ventil und ein zweites Ventil. Dabei ist die Pumpe ausgangsseitig mit dem ersten Volumen (auch Hochdruckvolumen genannt) verbunden, um so Gas in das erste Volumen hinein pumpen zu können. Das erste Volumen und das zweite Volumen (auch Niederdruckvolumen genannt) sind mittels des ersten Ventils miteinander verbunden, so dass abhängig von dem Zustand (geöffnet bzw. geschlossen) des Ventils das erste Volumen mit dem zweiten Volumen verbunden ist oder die beiden Volumen getrennt sind. Eingangsseitig ist die Pumpe zum einen über das zweite Ventil mit dem zweiten Volumen verbunden, so dass die Pumpe Gas aus dem zweiten Volumen herauspumpen kann, wenn das zweite Ventil geöffnet ist. Zum anderen ist die Pumpe eingangsseitig mit der zu entgasenden Einrichtung verbindbar, so dass die Pumpe Gas von oder aus der Einrichtung weg pumpen kann, wenn die Pumpe mit der Einrichtung verbunden ist.

Die erfindungsgemäße Vorrichtung ermöglicht vorteilhafterweise, dass bei der Entgasung das extrahierte Gas in beide Volumen gepumpt bzw. gefördert wird, so dass der Druck auf der Ausgangsseite der Pumpe nur geringfügig ansteigt. Nach der Entgasung kann dieselbe Pumpe das extrahierte Gas aus dem zweiten Volumen in das erste Volumen pumpen, wodurch sich der Druck des extrahierten Gases in dem ersten Volumen erhöht. Dadurch löst die vorliegende Erfindung die vorab gestellte Aufgabe.

Vorzugsweise ist das zweite Volumen bzw. Niederdruckvolumen zumindest fünfmal, besser zehnmal, noch besser 20-mal größer als das erste Volumen bzw. Hochdruckvolumen. Es ist allerdings auch möglich, dass (abhängig von der vorhandenen Gasmenge und der Pumpe) das zweite Volumen bzw. Niederdruckvolumen 100-mal oder gar 1000-mal größer als das erste Volumen bzw. Hochdruckvolumen ist.

Gemäß dieser Ausführungsform ist das Hochdruckvolumen deutlich kleiner als das Niederdruckvolumen. Das Volumen des Niederdruckvolumens ist dabei vorteilhafterweise so gewählt, dass der Druck auf der Ausgangsseite der Pumpe beim Entgasen nicht derart ansteigt, dass die Effizienz der Pumpe beeinträchtigt wird.

Bei einer beispielhaften Anwendung kann nur sehr wenig Gas (ca. 1 ml) zur Verfügung stehen. In diesem Fall kann das erste Volumen (Hochdruckvolumen) 0,5 ml und das zweite Volumen (Niederdruckvolumen) 10 ml groß sein.

Die absoluten Größen der Volumen hängen in der Regel davon ab, wie viel Gas gemessen werden soll bzw. kann (d.h. zur Verfügung steht). Dabei können die absoluten Größen für die Volumen auch von einem eingesetzten Sensor (zur Analyse des Gases) abhängen. Es ist durchaus möglich, dass die Volumen im µl-Bereich liegen (also deutlich kleiner als 1 ml sind). Auf der anderen Seite können auch mehrere Liter an Gas (z.B. bei Emissionsmessungen) auftreten, wobei die Volumen dann entsprechend größer (im Bereich von 1 l bis 20 l) gewählt werden.

Die Verhältnisse zwischen dem Gasvolumen (Volumen des zu analysierenden Gases bzw. Entgasungsvolumen) und dem ersten und zweiten Volumen sind im Wesentlichen ähnlich. Das zweite Volumen (Niederdruckvolumen) wird meist gleich oder größer als das Gasvolumen gewählt, während das erste Volumen (Hochdruckvolumen) deutlich kleiner ist. Die Verhältnisse können von der eingesetzten Pumpe abhängig sein. Beispielsweise kann beim Einsatz einer Hochdruckpumpe auch das zweite Volumen (Niederdruckvolumen) nur z.B. ein Zehntel des Gasvolumens betragen. In diesem Fall wäre also das zweite Volumen kleiner als das Gasvolumen.

Gemäß einer erfindungsgemäßen Ausführungsform öffnet die Vorrichtung mit Hilfe ihrer Steuereinrichtung das erste Ventil und schließt das zweite Ventil. Nachdem das erste Ventil geöffnet und das zweite Ventil geschlossen ist, steuert die Steuereinrichtung die Pumpe an, um Gas von der zu entgasenden Einrichtung in das erste Volumen und in das mit dem ersten Volumen über das erste Ventil verbundene zweite Volumen zu pumpen.

Indem das erste Ventil geöffnet ist, pumpt die Pumpe während des Entgasungsschritts das Gas in ein sehr großes Volumen, das der Summe aus dem ersten Volumen und dem zweiten Volumen entspricht. Aufgrund dieses großen Volumens steigt der Druck in diesem Volumen beim Entgasungsschritt vorteilhafterweise nahezu nicht an. Dadurch kann beim Entgasungsschritt sehr lange mit einem möglichst geringen Druck (idealerweise absolutem Vakuum) in dem ersten Volumen und einer sehr geringen Druckdifferenz zwischen dem ersten Volumen und dem zweiten Volumen gearbeitet werden. Dadurch können vorteilhafterweise Gase mit den unterschiedlichsten Lösungskoeffizienten aus der zu entgasenden Einrichtung extrahiert werden, so dass sich alles Gas lösen kann und das Verhältnis der extrahierten Gase auch dem Verhältnis der Gase in der Einrichtung (z.B. in dem Isolieröl) entspricht.

Gemäß einer erfindungsgemäßen Ausführungsform schließt die Vorrichtung mit Hilfe ihrer Steuereinrichtung das erste Ventil und öffnet das zweite Ventil. Nachdem das erste Ventil geschlossen und das zweite Ventil geöffnet ist, steuert die Steuereinrichtung die Pumpe an, um Gas von dem zweiten Volumen bzw. Niederdruckvolumen in das erste Volumen bzw. Hochdruckvolumen zu pumpen.

Da das zweite Ventil geöffnet ist, kann die Pumpe nach dem Entgasungsschritt im Komprimierungsschritt Gas aus dem Niederdruckvolumen in das Hochdruckvolumen pumpen, um dort den Druck zu erhöhen. Durch den höheren Druck wird die Konzentration der Gase erhöht, was eine anschließende Analyse begünstigt.

Gemäß einer erfindungsgemäßen Ausführungsform umfasst die Vorrichtung ein drittes Ventil. Über dieses dritte Ventil ist die zu entgasende Einrichtung mit dem Eingang der Pumpe verbunden.

Das dritte Ventil ermöglicht, dass die Vorrichtung dauerhaft (über das dritte Ventil) mit der zu entgasenden Einrichtung verbunden ist. Mit diesem dritten Ventil ist es sehr einfach, den Entgasungsschritt und den Komprimierungsschritt zu steuern. Beim Entgasungsschritt ist das dritte Ventil geöffnet, so dass die Pumpe Gas von der zu entgasenden Einrichtung wegpumpen kann. Dagegen ist das dritte Ventil im Komprimierungsschritt geschlossen, so dass die Pumpe nur Gas von dem zweiten Volumen (und kein weiteres Gas von der Einrichtung) in das erste Volumen pumpt. Dies wird in den folgenden Ausführungsformen noch genauer beschrieben.

Gemäß einer erfindungsgemäßen Ausführungsform öffnet die Vorrichtung mit Hilfe ihrer Steuereinrichtung das erste Ventil und das dritte Ventil und schließt das zweite Ventil. Nachdem das erste Ventil und das dritte Ventil geöffnet sind und das zweite Ventil geschlossen ist, steuert die Steuereinrichtung die Pumpe an, um Gas von der zu entgasenden Einrichtung in das erste Volumen und in das mit dem ersten Volumen über das erste Ventil verbundene zweite Volumen zu pumpen.

Da das erste Ventil und das dritte Ventil geöffnet sind, pumpt die Pumpe während des Entgasungsschritts das Gas in das erste Volumen und das über das erste Ventil mit dem ersten Volumen verbundene zweite Volumen. Dadurch kann beim Entgasungsschritt sehr lange mit einem sehr geringen Druck in dem ersten und zweiten Volumen gearbeitet werden, wodurch sich vorteilhafterweise alle Gase in der Einrichtung lösen können.

Gemäß einer erfindungsgemäßen Ausführungsform schließt die Vorrichtung mit Hilfe ihrer Steuereinrichtung das erste Ventil und das dritte Ventil und öffnet das zweite Ventil. Nachdem das erste Ventil und das dritte Ventil geschlossen sind und das zweite Ventil geöffnet ist, steuert die Steuereinrichtung die Pumpe an, um Gas von dem zweiten Volumen bzw. Niederdruckvolumen in das erste Volumen bzw. Hochdruckvolumen zu pumpen.

Da nur das zweite Ventil geöffnet ist und das erste und das dritte Ventil geschlossen sind, pumpt die Pumpe im Komprimierungsschritt nur Gas aus dem Niederdruckvolumen in das Hochdruckvolumen. Da das dritte Ventil geschlossen ist, wird dabei kein Gas von der Einrichtung von der Pumpe angezogen, und da auch das erste Ventil geschlossen ist, wird dabei auch kein Gas von der Pumpe (über das Niederdruckvolumen) aus dem Hochdruckvolumen angezogen.

Gemäß einer erfindungsgemäßen Ausführungsform umfasst die Vorrichtung einen Sensor. Dabei ist der Sensor zumindest teilweise innerhalb des ersten Volumens angeordnet und ausgestaltet, um ein Gas in dem ersten Volumen zu analysieren.

Mit Hilfe des Sensors kann die Analyse des Gases in dem ersten Volumen bzw. Hochdruckvolumen nach dem Komprimierungsschritt quasi automatisiert erfolgen. Dieser Sensor misst die Gase. Bei dem Sensor kann es sich beispielsweise um einen Halbleitersensor, einen optischen Sensor (oder eine optische Messapparatur), einen Wärmeleitfähigkeitssensor oder um einen chemischen Analyseapparat (z.B. einen Gaschromatograph) handeln. Mit anderen Worten ist der Sensor insbesondere irgendeine Vorrichtung, die Gase messen kann und - in diesem Fall - diese Gase in einer möglichst hohen (absoluten) Konzentration benötigt.

Die erfindungsgemäße Vorrichtung ermöglicht vorteilhafterweise, dass der Entgasungsschritt und der Komprimierungsschritt mit nur einer bzw. derselben Pumpe ausgeführt werden. Beim Komprimierungsschritt kann der Druck in dem Hochdruckvolumen so lange erhöht werden, bis ein gewünschter Druckwert erreicht ist, alles Gas aus dem Niederdruckvolumen gepumpt ist oder die Kapazität der Pumpe erschöpft ist. Durch den Einsatz nur einer Pumpe sowohl zum Entgasen als auch zum Komprimieren werden vorteilhafterweise der Raumbedarf sowie die Kosten für eine weitere Pumpe eingespart.

Selbst wenn im Komprimierungsschritt nicht alles Gas von dem Niederdruckvolumen in das Hochdruckvolumen gepumpt wird, bleibt vorteilhafterweise das Verhältnis der Gase das gleiche wie das Verhältnis der Gase in der Einrichtung (z.B. in dem Isolieröl). Dies wäre beispielsweise nicht der Fall, wenn die Pumpe das Gas in dem Komprimierungsschritt direkt aus der Einrichtung in das Hochdruckvolumen pumpen würde. Darüber hinaus kann beim Komprimierungsschritt ein höherer Druck im Hochdruckvolumen erzeugt werden als beim direkten Entgasen (ohne ein Niederdruckvolumen), da der Eingangsdruck höher ist.

Im Rahmen der vorliegenden Erfindung wird auch ein Prüfsystem zum Prüfen von gelösten Gasen und Gas an oder in einer Anlage, wie z.B. einer Hochspannungsanlage, bereitgestellt. Dabei umfasst das Prüfsystem eine Auswerteeinheit und eine erfindungsgemäße Vorrichtung zur Entgasung, wie sie vorab beschrieben ist. Das Prüfsystem ist ausgestaltet, um eine Analyse des Gases in oder von der Anlage (z.B. eine Analyse des in dem Isolieröl einer Hochspannungsanlage gelösten Gases) auszuführen. Dabei ist die Auswerteeinheit ausgestaltet, um, beispielsweise mit Hilfe des Sensors der Vorrichtung, das in das erste Volumen gepumpte Gas zu analysieren und um abhängig von dieser Analyse ein Ergebnis der Prüfung der Anlage zu erstellen und vorteilhafterweise auszugeben.

Das erfindungsgemäße Prüfsystem kann in ähnlicher Weise wie die erfindungsgemäße Vorrichtung an ölisolierten Hochspannungsanlagen, wie z.B. Leistungstransformatoren, Stromwandlern, Spannungswandlern und mittels Gas isolierten Schaltanlagen, eingesetzt werden. Bei dem zu analysierenden Gas kann es sich um ein Gas, welches zur Isolierung der Hochspannungsanlage selbst eingesetzt wird, oder um ein Gas, welches sich aus einer Flüssigkeit einer Isolierung oder einem Isolieröl gelöst hat, handeln.

Schließlich wird im Rahmen der vorliegenden Erfindung ein Verfahren zur Entgasung einer Einrichtung bereitgestellt. Dieses Verfahren umfasst folgende Schritte:
- Verbinden eines ersten Volumens mit einem zweiten Volumen. Dieser Schritt kann beispielsweise ausgeführt werden, indem ein Ventil zwischen dem ersten und dem zweiten Volumen geöffnet wird. Dieser Schritt dient quasi der Vorbereitung des folgenden Schrittes.
- Pumpen eines Gases von der Einrichtung in das erste Volumen und damit in das zweite Volumen, das im vorherigen Schritt mit dem ersten Volumen verbunden wurde. In diesem Schritt wird das Gas von der Einrichtung in ein großes Volumen gepumpt, das sich aus dem ersten und dem zweiten Volumen zusammensetzt. Ein großes Volumen erleichtert vorteilhafterweise das Entgasen.
- Trennen der Verbindung zwischen dem ersten Volumen und dem zweiten Volumen. Dieser Schritt kann beispielsweise ebenfalls mit Hilfe des im ersten Schritt genannten Ventils ausgeführt werden, indem dieses Ventil jetzt geschlossen wird.
- Pumpen des Gases von dem zweiten Volumen in das erste Volumen. In diesem Schritt wird das Gas komprimiert, indem der Teil des von der Einrichtung entzogenen Gases, welcher sich in dem zweiten Volumen befindet, in das erste Volumen gepumpt wird.

Die Vorteile des erfindungsgemäßen Verfahrens entsprechen im Wesentlichen den Vorteilen der erfindungsgemäßen Vorrichtung, welche vorab beschrieben sind, so dass hier auf eine Wiederholung verzichtet wird.

Bei der zu entgasenden Einrichtung handelt es sich insbesondere um ein Entgasungsgefäß, in das beispielsweise manuell eine zu entgasende Flüssigkeit (z.B. Öl) gefüllt wird. Es ist allerdings auch möglich, dass kein manueller Vorgang erforderlich ist, indem beispielsweise die zu entgasende Flüssigkeit automatisch in das Entgasungsgefäß gefüllt (gepumpt) wird oder indem das der erfindungsgemäßen Vorrichtung oder dem erfindungsgemäßen Prüfsystem zugeführte und zu analysierende Gas direkt einer Anlage (z.B. einer Hochspannungsanlage) entnommen wird. In dem letzteren Fall entspricht die zu entgasende Einrichtung quasi der Anlage.

Neben der Überprüfung von Hochspannungsanlagen kann die vorliegende Erfindung auch allgemein für Gasmessgeräte eingesetzt werden. Damit kann die vorliegende Erfindung zur Qualitätskontrolle im Labor, zur Prozessanalyse und Prozessüberwachung eingesetzt werden für:
- Petrochemie- und Chemie-Anlagen
- Erdgas-Aufbereitungsanlagen
- Bio-Gas-Anlagen
- Brennwertbestimmungen bei online Erdgasanalysen und bei Energieerzeugungen
- Emissionsmessungen

### KURZE BESCHREIBUNG DER FIGUREN

Die Erfindung wird nachfolgend anhand bevorzugter Ausführungsformen und unter Bezugnahme auf die Zeichnungen näher erläutert.
In Fig. 1 ist schematisch eine erfindungsgemäße Vorrichtung dargestellt, welche mit einem zu entgasenden Entgasungsgefäß verbunden ist.
In Fig. 2 ist schematisch ein Prüfsystem dargestellt, welches mit einer zu prüfenden Hochspannungsanlage verbunden ist.

### DETAILLIERTE BESCHREIBUNG VON AUSFÜHRUNGSBEISPIELEN

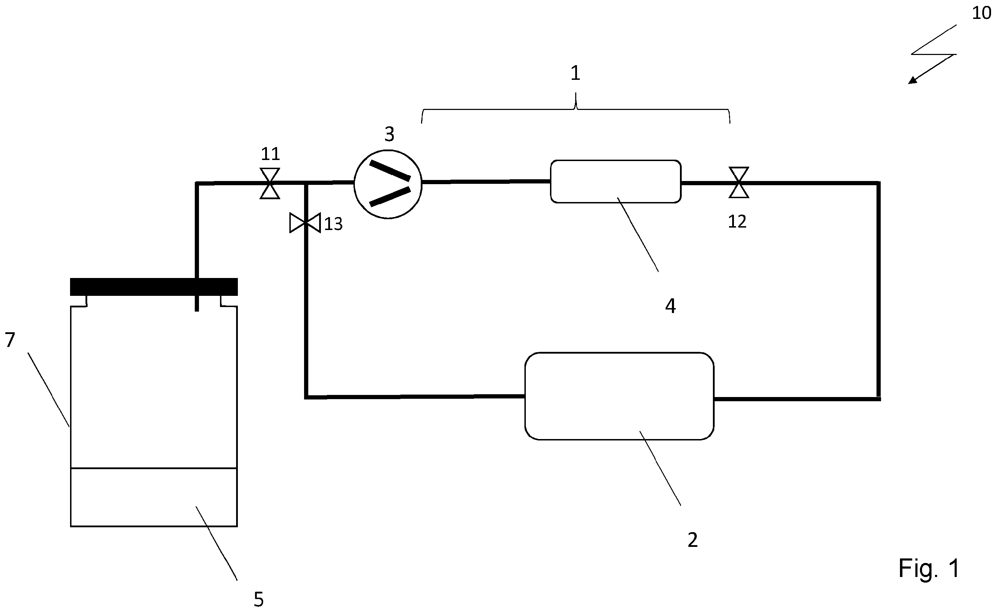

In Fig. 1 ist schematisch eine erfindungsgemäße Vorrichtung 10 dargestellt, welche mit einem Entgasungsvolumen bzw. Entgasungsgefäß 7 in Verbindung steht.

Die Vorrichtung umfasst ein erstes Ventil 12, ein zweites Ventil 12, ein drittes Ventil 11, eine Pumpe 3, ein erstes Gefäß bzw. Volumen 1, ein zweites Gefäß bzw. Volumen 2 und einen Sensor 4, der in dem ersten Volumen 1 angeordnet ist. Das erste Ventil 12 ist zwischen dem ersten Volumen 1 und dem zweiten Volumen 2 angeordnet. Das heißt, dass das erste Volumen 1 und das zweite Volumen 2 quasi ein großes Volumen bilden, wenn das erste Ventil 12 geöffnet ist. Die Pumpe 3 ist so angeordnet, dass die Pumpe eingangsseitig über das zweite Ventil 13 mit dem Entgasungsgefäß 7 und/oder über das dritte Ventil 11 mit dem zweiten Volumen 2 in Verbindung steht.

Beispielweise kann das Entgasungsvolumen (Volumen des Entgasungsgefäßes 7) einen Volumeninhalt von ca. 300 ml (ca. 700 ml) (abhängig von dem verwendeten Entgasungsgefäß 7), das zweite Volumen 2 (Niederdruckvolumen oder Entspannungsvolumen) einen Volumeninhalt von ca. 10 ml und das erste Volumen 1 (Hochdruckvolumen oder Analysevolumen) einen Volumeninhalt von ca. 500 µl aufweisen.

In dem Entgasungsgefäß 7 befindet sich Isolieröl 5. Die Analyse von in diesem Isolieröl 5 gelösten Gasen wird mit Hilfe des Sensors 4 vorgenommen, indem diese aus dem Isolieröl gelösten Gase in das erste Volumen 1 gepumpt und dort mit Hilfe des Sensors 4 analysiert werden.

Dazu werden in dem Entgasungsschritt das erste Ventil 12 und das dritte Ventil 11 geöffnet und das zweite Ventil 13 geschlossen. Dann pumpt die Pumpe 3 die Gase aus dem Entgasungsgefäß 7 in das erste Volumen 1 und von dort über das erste Ventil 12 in das zweite Volumen 2. Anschließend werden das erste Ventil 12 und das dritte Ventil 11 geschlossen und das zweite Ventil 13 geöffnet. Dann pumpt die Pumpe 3 die Gase aus dem zweiten Volumen 2 in das erste Volumen 1, wodurch der Druck in dem ersten Volumen 1 und damit die Konzentration der Gase in dem ersten Volumen 1 ansteigt. Wenn ein möglichst maximaler Druck in dem ersten Volumen 1 erreicht ist, werden die Gase in dem ersten Volumen 1 mit Hilfe des Sensors 4 analysiert.

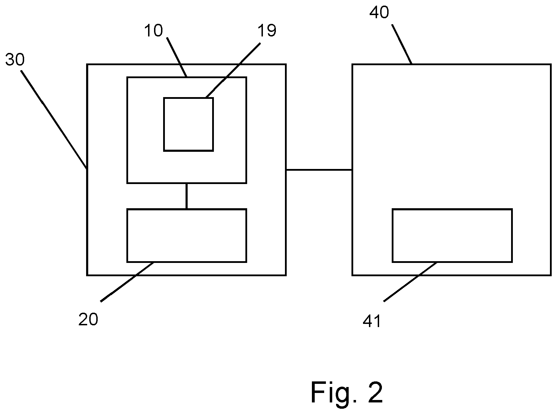

Die Durchführung des Entgasungsvorgangs und die Ansteuerung der Ventile 11-13 sowie der Pumpe 3 erfolgt vorzugsweise automatisch bzw. rechnergestützt mittels einer geeigneten Steuereinrichtung (vgl. die in Fig. 2 gezeigte Steuereinrichtung 19).

In Fig. 2 sind schematisch ein erfindungsgemäßes Prüfsystem 30 und eine Hochspannungsanlage 40 dargestellt. Dabei ist das Prüfsystem 30 ausgestaltet, um eine Isolierung 41 der Hochspannungsanlage 40 zu überprüfen. Das Prüfsystem 30 umfasst eine erfindungsgemäße Vorrichtung 10 zur Entgasung, wie sie vorab beschrieben und in Fig. 1 schematisch dargestellt ist. Darüber hinaus umfasst das Prüfsystem 30 eine Auswerteeinheit 20, um abhängig von der durch die Vorrichtung 10 mit ihrer Steuereinrichtung 19 ausgeführten Entgasung, Komprimierung und anschließenden Analyse des Gases ein Ergebnis der Überprüfung zu erstellen. Dabei analysiert die Vorrichtung 10 ein aus der Isolierung 41 kommendes Gas, wobei anhand der Analyse dieses Gases die Qualität der Isolierung 41 und damit ein Maß für die Einsatzbereitschaft der Hochspannungsanlage 40 selbst bestimmt werden kann.

## Patentansprüche

1. Vorrichtung zur Entgasung einer Einrichtung (7),
mit einer Pumpe (3), die ausgangsseitig mit einem ersten Volumen (1) verbunden ist, wobei das erste Volumen (1) mit einem zweiten Volumen (2) über ein erstes Ventil (12) verbunden ist,
wobei die Pumpe (3) eingangsseitig zum einen über ein zweites Ventil (13) mit dem zweiten Volumen (2) verbunden ist und zum anderen mit der zu entgasenden Einrichtung (7) verbindbar ist, und
mit einer Steuereinrichtung (19) zum Ansteuern der Pumpe (3) und der ersten und zweiten Ventile (12, 13),
**dadurch gekennzeichnet,**
**dass** die Vorrichtung (10) ausgestaltet ist, um mittels der Steuereinrichtung (19) das erste Ventil (12) zu öffnen und das zweite Ventil (13) zu schließen und um anschließend mittels der Pumpe (3) Gas von der zu entgasenden Einrichtung (7) in das erste Volumen (1) und in das mit dem ersten Volumen (1) verbundene zweite Volumen (2) zu pumpen.

2. Vorrichtung nach Anspruch 1,
**dadurch gekennzeichnet,**
**dass** das zweite Volumen (2) zumindest fünfmal größer als das erste Volumen (1) ist.

3. Vorrichtung nach einem der vorhergehenden Ansprüche,
**dadurch gekennzeichnet,**
**dass** die Vorrichtung (10) ausgestaltet ist, um mittels der Steuereinrichtung (19) das erste Ventil (12) zu schließen und das zweite Ventil (13) zu öffnen und um anschließend mittels der Pumpe (3) Gas von dem zweiten Volumen (2) in das erste Volumen (1) zu pumpen.

4. Vorrichtung nach einem der vorhergehenden Ansprüche,
**dadurch gekennzeichnet,**
**dass** die Vorrichtung (10) ein drittes Ventil (11) umfasst, mit welchem die Pumpe (3) eingangsseitig mit der zu entgasenden Einrichtung (7) verbunden ist.

5. Vorrichtung nach Anspruch 4,
**dadurch gekennzeichnet,**
**dass** die Vorrichtung (10) ausgestaltet ist, um mittels der Steuereinrichtung (19) das erste Ventil (12) und das dritte Ventil (11) zu öffnen und das zweite Ventil (13) zu schließen und um anschließend mittels der Pumpe (3) Gas von der zu entgasenden Einrichtung (7) in das erste Volumen (1) und in das mit dem ersten Volumen (1) verbundene zweite Volumen (2) zu pumpen.

6. Vorrichtung nach Anspruch 4 oder 5,
**dadurch gekennzeichnet,**
**dass** die Vorrichtung (10) ausgestaltet ist, um mittels der Steuereinrichtung (19) das erste Ventil (12) und das dritte Ventil (11) zu schließen und das zweite Ventil (13) zu öffnen und um anschließend mittels der Pumpe (3) Gas von dem zweiten Volumen (2) in das erste Volumen (1) zu pumpen.

7. Vorrichtung nach einem der vorhergehenden Ansprüche,
**dadurch gekennzeichnet,**
**dass** die Vorrichtung (10) einen Sensor (4) umfasst, welcher in dem ersten Volumen (1) angeordnet ist, und
**dass** der Sensor (4) ausgestaltet ist, um das Gas in dem ersten Volumen (1) zu analysieren.

8. Vorrichtung nach einem der vorhergehenden Ansprüche,
**dadurch gekennzeichnet,**
**dass** die Vorrichtung (10) zur Entgasung einer Hochspannungsanlage (40) ausgestaltet ist.

9. Prüfsystem zum Prüfen einer Einrichtung (7),
wobei das Prüfsystem (30) eine Auswerteeinheit (20) und eine Vorrichtung (10) nach einem der vorhergehenden Ansprüche umfasst, um eine Analyse des Gases in oder von der Einrichtung (7) durchzuführen,
wobei die Auswerteeinheit (20) ausgestaltet ist, um das in das erste Volumen (1) gepumpte Gas zu analysieren und um abhängig von der Analyse ein Ergebnis der Prüfung der Einrichtung (7) zu erstellen.

10. Verfahren zur Entgasung einer Einrichtung (7) mit folgenden Schritten:
Verbinden eines ersten Volumens (1) mit einem separat von dem ersten Volumen (1) vorgesehenen zweiten Volumen (2),
Pumpen eines Gases von der Einrichtung (7) in das erste Volumen (1) und von dort in das mit dem ersten Volumen (1) verbundene zweite Volumen (2),
Abtrennen des zweiten Volumens (2) von dem ersten Volumen (1), und
Pumpen des Gases aus dem zweiten Volumen (2) in das erste Volumen (1),
**dadurch gekennzeichnet, dass** das Verfahren mit einer Vorrichtung (10) nach einem der Ansprüche 1-8 ausgeführt wird.

## Claims

1. An apparatus for degassing a device (7),
having a pump (3), which, at a discharge end, is connected to a first volume (1),
wherein the first volume (1) is connected to a second volume (2) via a first valve (12),
wherein, at an intake end, the pump (3) is connected to the second volume (2) via a second valve (13) and can be connected to the device (7) to be degassed, and
having a control unit (19) for activating the pump (3) and the first and second valves (12, 13),
**characterised in that**
the apparatus (10) is designed in order to open the first valve (12) and close the second valve (13) by means of the control unit (19) and to then pump gas, from the device (7) to be degassed, into the first volume (1) and into the second volume (2), which is connected to the first volume (1), by means of the pump (3).

2. The apparatus according to claim 1,
**characterised in that**
the second volume (2) is at least five times larger than the first volume (1).

3. The apparatus according to one of the preceding claims,
**characterised in that**
the apparatus (10) is designed in order to close the first valve (12) and open the second valve (13) by means of the control unit (19) and to then pump gas from the second volume (2) into the first volume (1) by means of the pump (3).

4. The apparatus according to one of the preceding claims,
**characterised in that**
the apparatus (10) comprises a third valve (11), with which, at the intake end, the pump (3) is connected to the device (7) to be degassed.

5. The apparatus according to claim 4,
**characterised in that**
the apparatus (10) is designed in order to open the first valve (12) and the third valve (11) and close the second valve (13) by means of the control unit (19) and in order to then pump gas from the device (7) to be degassed into the first volume (1), and into the second volume (2) connected to the first volume (1), by means of the pump (3).

6. The apparatus according to claim 4 or 5,
**characterised in that**
the apparatus (10) is designed in order to close the first valve (12) and the third valve (11) and to open the second valve (13) by means of the control unit (19) and to then pump gas from the second volume (2) into the first volume (1) by means of the pump (3).

7. The apparatus according to one of the preceding claims,
**characterised in that**
the apparatus (10) comprises a sensor (4), which is arranged in the first volume (1), and
the sensor (4) is designed in order to analyze the gas in the first volume (1).

8. The apparatus according to one of the preceding claims,
**characterised in that**
the apparatus (10) is designed for degassing a high-voltage installation (40).

9. A test system for testing a device (7),
wherein the test system (30) comprises an evaluation unit (20) and an apparatus (10) according to one of the preceding claims, in order to carry out an analysis of the gas in or from the device (7),
wherein the evaluation unit (20) is designed in order to analyze the gas pumped into the first volume (1) and, depending on the analysis, to draw up a result of the testing of the device (7).

10. A method of degassing a device (7) having the following steps:
connecting a first volume (1) to a second volume (2) which is provided separately from the first volume (1),
pumping a gas from the device (7) into the first volume (1) and from there into the second volume (2) which is connected to the first volume (1),
disconnecting the second volume (2) from the first volume (1) and
pumping the gas out of the second volume (2) into the first volume (1),
**characterised in that**
the method is performed with an apparatus (10) according to one of claims 1-8.

## Revendications

1. Dispositif de dégazage d'un appareil (7),
comprenant une pompe (3) reliée en sortie à un premier volume (1), le premier volume (1) étant relié à un deuxième volume (2) par une première vanne (12),
la pompe (3) étant reliée en entrée, d'une part, au deuxième volume (2) par une deuxième vanne (13) et étant susceptible d'être reliée, d'autre part, à l'appareil (7) à dégazer, et
comprenant un organe de commande (19) pour commander la pompe (3) et les première et deuxième vannes (12, 13),
**caractérisé en ce que**
le dispositif (10) est conçu pour ouvrir la première vanne (12) et fermer la deuxième vanne (13) au moyen de l'organe de commande (19), puis pour pomper le gaz de l'appareil (7) à dégazer vers le premier volume (1) et vers le deuxième volume (2), relié au premier volume (1), au moyen de la pompe (3).

2. Dispositif selon la revendication 1,
**caractérisé en ce que**
le deuxième volume (2) est au moins cinq fois plus grand que le premier volume (1).

3. Dispositif selon l'une des revendications précédentes,
**caractérisé en ce que**
le dispositif (10) est conçu pour fermer la première vanne (12) et ouvrir la deuxième vanne (13) au moyen de l'organe de commande (19), puis pour pomper le gaz du deuxième volume (2) vers le premier volume (1) au moyen de la pompe (3).

4. Dispositif selon l'une des revendications précédentes,
**caractérisé en ce que**
le dispositif (10) comprend une troisième vanne (11) par laquelle la pompe (3) est reliée en entrée à l'appareil (7) à dégazer.

5. Dispositif selon la revendication 4,
**caractérisé en ce que**
le dispositif (10) est conçu pour ouvrir la première vanne (12) et la troisième vanne (11) et fermer la deuxième vanne (13) au moyen de l'organe de commande (19), puis pour pomper le gaz de l'appareil (7) à dégazer vers le premier volume (1) et vers le deuxième volume (2), relié au premier volume (1), au moyen de la pompe (3).

6. Dispositif selon la revendication 4 ou 5,
**caractérisé en ce que**
le dispositif (10) est conçu pour fermer la première vanne (12) et la troisième vanne (11) et ouvrir la deuxième vanne (13) au moyen de l'organe de commande (19), puis pour pomper le gaz du deuxième volume (2) vers le premier volume (1) au moyen de la pompe (3).

7. Dispositif selon l'une des revendications précédentes,
**caractérisé en ce que**
le dispositif (10) comprend un capteur (4) disposé dans le premier volume (1), et
**en ce que** le capteur (4) est conçu pour analyser le gaz présent dans le premier volume (1).

8. Dispositif selon l'une des revendications précédentes,
**caractérisé en ce que**
le dispositif (10) est conçu pour dégazer un système haute tension (40).

9. Système de test pour tester un appareil (7),
le système de test (30) comprenant une unité d'évaluation (20) et un dispositif (10) selon l'une des revendications précédentes pour effectuer une analyse du gaz présent dans ou provenant de l'appareil (7),
l'unité d'évaluation (20) étant conçue pour analyser le gaz pompé dans le premier volume (1) et pour générer un résultat de test de l'appareil (7) en fonction de ladite analyse.

10. Procédé de dégazage d'un appareil (7), comprenant les étapes suivantes consistant à :
relier un premier volume (1) à un deuxième volume (2) prévu séparément du premier volume (1),
pomper un gaz de l'appareil (7) vers le premier volume (1) et de là vers le deuxième volume (2) relié au premier volume (1),
séparer le deuxième volume (2) du premier volume (1), et
pomper le gaz du deuxième volume (2) vers le premier volume (1),
**caractérisé en ce que** le procédé est mis en œuvre à l'aide d'un dispositif (10) selon l'une des revendications 1 à 8.
